# EUROPEAN PATENT APPLICATION

(11) **EP 3 544 206 A1**
(43) Date of publication of application: **25.09.2019**
(21) Application number: 18162738.1
(22) Date of filing: 20.03.2018
(51) Int. Cl.: H04B 7/08, H04B 7/10, A61B 5/00, A61N 1/372

(54) **IMPROVING ANTENNA COUPLING OF IMPLANTABLE DEVICES OR DEVICES ATTACHED TO A PERSON AND AN EXTERNAL DEVICE BY ORIENTATION DEPENDENT SWITCHING OF ANTENNAS**

(71) Applicant: BIOTRONIK SE & Co. KG, 12359 Berlin (DE)
(72) Inventor: Ulmer, Jens, 8700 Küsnacht (CH); Finnberg, Thomas, 8037 Zürich (CH)
(74) Representative: Keck, Hans-Georg

(57) **Abstract**

The present invention relates to a system (1), comprising: a first device (10) having at least two antennas (11a, 11b) for transmitting and/or receiving a radio signal. According to the present invention, the first device (10) comprises a sensor (12) that is configured to generate an output signal indicative of a spatial orientation of the first device (10), wherein the first device (10) is configured to use the output signal to select one of said antennas (11a, 11b) or to control said antennas (11a, 11b) for receiving a radio signal (C) and/or for transmitting a radio signal (C).

## Description

The present invention relates to a system, particularly a medical system, comprising at least first device having at least two antennas for transmitting and/or receiving a radio signal.

For transmitters/receivers that are either implanted or worn close to the body, the transmission and reception properties change depending on the position of the patient wearing the transmitter/receiver in relation to an external radio device that interacts with the transmitter/receiver on the person.

Particularly, WO2015196164A2 describes multiples antennas for an implant, wherein the antennas are arranged differently to make the link gain less sensible to the relative alignment to an external transceiver.

Based on the above, it is an objective of the present invention to provide an improved radio communication between a first device and a second device that is less sensitive to the orientation of the first device with respect to the second device.

This problem is solved by a system having the features of claim 1. Embodiments of the present invention are stated in the sub claims and are described below.

According to the present invention, the first device comprises a sensor that is configured to generate an output signal indicative of a spatial orientation of the first device (e.g. with respect to a reference direction or with respect to a second device), wherein the first device is configured to use the output signal to select one of said antennas or to control said antennas for receiving a radio signal (e.g. from the second device of the system) and/or for transmitting a radio signal (e.g. to said second device).

Particularly, the invention thus allows to monitor a patient, for example at the sickbed, via a wireless connection that functions reliably. This is advantageous since the body of a patient tends to partly absorb or reflects high-frequency signals in certain positions of the patient. Particularly, the switching of antennas depending on spatial orientation according to the present invention allows to improve antenna coupling and to maintain a functioning communication.

Particularly, the spatial orientation of the first device (and particularly of the associated antennas) is defined by three parameters corresponding to the three degrees of freedom of the spatial orientation. The parameters can be three angles such as azimuth, pitch and roll.

According to an embodiment of the system according to the present invention, the system comprises a second device configured to transmit a radio signal to the first device and/or to receive a radio signal from the first device.

Furthermore, according to an embodiment of the system according to the present invention, the first device is configured to select the antenna of the at least two antennas of the first device having the highest coupling with respect to at least one antenna of the second device, particularly so as to maintain a stable radio communication for different spatial orientations of the first and the second device with respect to each other.

Further, according to an embodiment of the system according to the present invention, the at least two antennas of the first device each comprise an antenna polarization, wherein said antenna polarizations are different, particularly orthogonal with respect to each other.

Further, according to an embodiment of the system according to the present invention, the at least two antennas of the first device are arranged at different locations.

Furthermore, according to an embodiment of the system according to the present invention, the first device is configured to control the at least two antennas of the first device in a phase-controlled manner in order to increase the coupling between said at least two antennas of the first device and at least one antenna of the second device. By using a phased controlled antenna excitation the effective antenna gain can be improved leading to lower antenna coupling loss between the two devices.

Furthermore, according to an embodiment, the first device is a medical device configured to be implanted into a patient or to be arranged on a patient.

Further, in an embodiment, the second device is an external device that can e.g. be configured to receive radio signals from the first device or to transmit radio signals to the first device. Particularly, the notion external in this regard means that the second device is arranged in a surrounding of the patient outside the patient, particularly remote from the patient.

However, alternatively, it is also possible that the first device is the external device that can e.g. be configured to receive radio signals from a second device or to transmit radio signals to the second device. Here, the external device comprises the sensor and at least two antennas. Further, in this case, according to an embodiment, the second device can be the medical device configured to be implanted into a patient or to be arranged on the patient. For instance, the second device can be configured to transmit radio signals with position information regarding the position of the second device and/or the position of the patient to the external device. The signals are received by the external device, whereupon the external device selects one of the at least two antennas for transmitting radio signals to the second device. The antenna is selected depending on the position information.

Further, according to an embodiment, both devices, i.e. the first and the second device can each comprise a sensor for measuring the respective spatial orientations as well as at least two antennas, wherein particularly the respective device is configured to select or control its antennas based on the output signal of the respective sensor, and particularly also depending on the output signal of the respective other sensor.

Particularly, according to an embodiment, the system (e.g. the first device) can be configured to also use said output signal of the sensor of the second device to select one of said antennas of the first device or to control said antennas of the first device for receiving a radio signal and/or for transmitting a radio signal (see also above).

Particularly, according to an embodiment of the present invention, the present invention can be applied to all kind of (e.g. medical) devices. Particularly, the implantable medical device is a cardiac pacemaker, a neuro stimulating device or a monitoring device that records patient data and/or transmits patient data to the external device.

Furthermore, in case of a medical device that is configured to be arranged on the patient (from the outside), the medical device can be formed as a chest belt or may comprise a chest belt, wherein said chest belt is configured to be fastened to a chest of the patient. Here, particularly, said at least two antennas can be arranged at different locations along the chest belt and/or may comprise different, particularly orthogonal polarizations. Such a medical device having a chest belt can be a monitoring device, e.g. for sensing an electrocardiogram or other physiological data of the patient.

Moreover, the medical device 10 can include a patch system which is attachable to the patient. According to embodiments of the invention, the medical device can 10 can be worn by the patient in form of a belt, a watch, eyeglasses, a headband or any clothing item.

Further, according to an embodiment of the present invention, the external device can be configured to control the first device and/or to communicate with the first device, particularly via radio signals. Particularly, the external device can be or comprise a remote control and/or data storage for storing patient data transmitted by the (e.g. implantable) medical device.

The respective antenna of the first and/or second device can be a dipole antenna or a monopole antenna. Furthermore, in all embodiments described above, the respective external device can be a mobile device, particularly a hand-held device, or any stationary device, e.g. a wall mounted devices/antennas.

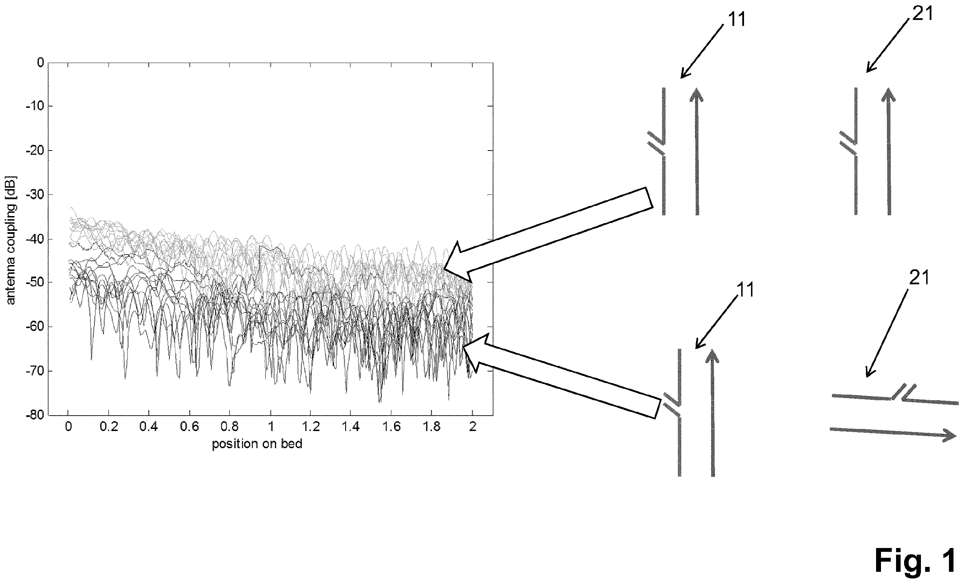

Further features, advantages and embodiments of the present invention will be described in the following with reference to the figures, wherein
- Fig. 1: shows a coupling of two antennas, e.g. a transmitting antenna and a receiving antenna, for two different orientations of the antennas with respect to each other;

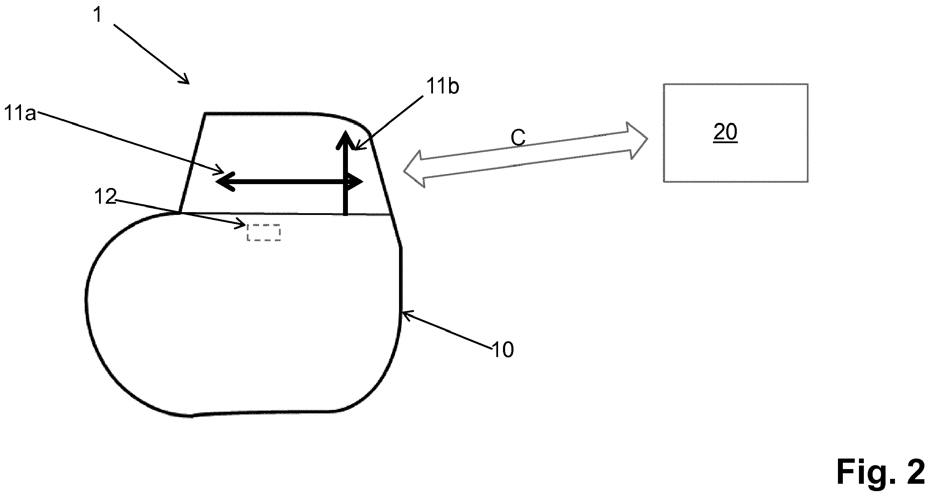

- Fig. 2: shows an embodiment of a system according to the present invention comprising a first device in the form of an implantable medical device (e.g. cardiac pacemaker) and an external second device, wherein the implantable medical device comprises at least two antennas and a sensor for sensing a spatial orientation of the implantable medical device;

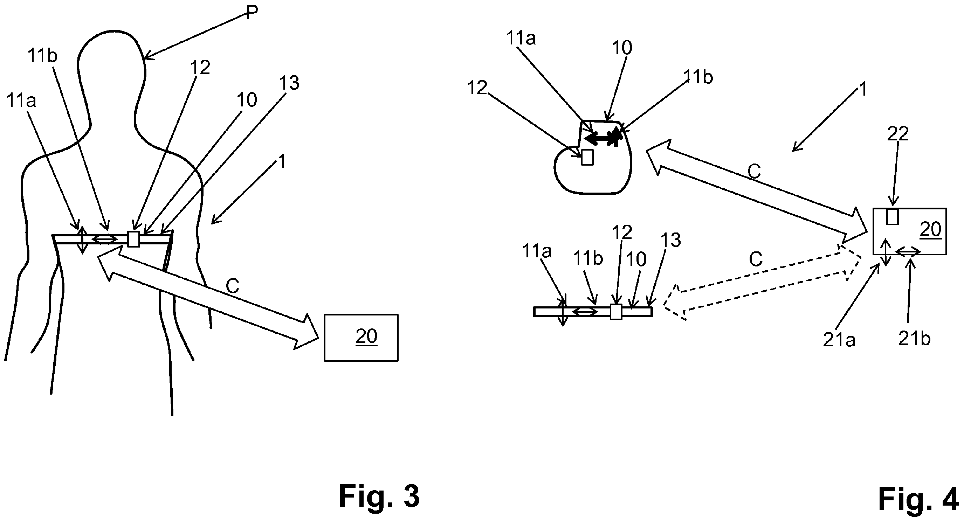

- Fig. 3: shows a further embodiment of a system according to the present invention comprising a first device in the form of a medical device having a chest belt or patch to be arranged on the patient (e.g. on the skin of the patient) and an external second device, wherein the medical device comprises at least two antennas and a sensor for sensing a spatial orientation of the medical device; and
- Fig. 4: shows a variant of the embodiments shown in Figs. 2 and 3, wherein here also the external second device comprises a sensor for sensing a spatial orientation of the external device and at least two antennas for sending radio signals to the medical device or for receiving radio signals from the medical device.

According to an embodiment of a system 1 according to the present invention as shown in Fig. 2, the system 1 comprises at least a first device 10 having at least two antennas 11a, 11b for transmitting and/or receiving a radio signal C (e.g. to a second device 20 of the system), wherein the first device 10 comprises a sensor 12 that is configured to generate an output signal indicative of a spatial orientation of the first device 10 (e.g. with respect to some reference direction or with respect to the second device 20), wherein the first device 10 is configured to use the output signal to select one of said antennas 11a, 11b or to control said antennas 11a, 11b for receiving a radio signal C (e.g. from said second device 20) and/or for transmitting a radio signal C (e.g. to said second device 20).

Particularly, the first device 10 can be an implantable medical device 10 such as a cardiac pacemaker 10. According to an embodiment, an antenna 11a of said two antennas 11a, 11b can be a dipole antenna 11a whereas the other antenna 11b can be a monopole antenna. The antennas 11a, 11b particularly comprise different, particularly orthogonal polarizations as indicated in Fig. 2.

Thus, using the output signal of the sensor 12, the first device 10 is configured to use the antenna 11a or 11b that - due to its spatial orientation - comprises a higher coupling with an antenna of the external second device 20 which can be a remote control device and/or a device for recording data provided by the medical implant 10.

Alternatively, depending on said output signal, the first device 10 may also be configured to control the at least two antennas 11a, 11b of the first device 10 in a phase-controlled manner in order to increase a coupling between said at least two antennas 11a, 11b of the first device 10 and at least one antenna of the second device 20.

Thus, in the embodiment shown in Fig. 2 the implantable medical device 10 particularly uses the sensor 12 to determine the current spatial orientation with respect to the external transmitting or receiving device 20. In case the spatial position of the implantable medical device 10 changes relative to the external device 20, the most suitable antenna 11a, 11b is selected based on the output signal of the sensor 12 which is indicative of said spatial orientation. Here, particularly, the notion most suitable can mean that the antenna 11a, 11b having the best transmitting and/or receiving characteristics with respect to the external device 20.

In some cases the orientation of the external device is defined by its design or by the operating manual and is therefore known. If this is not the case the external device 20 has to estimates it's spatial orientation with its sensor 22 and transmit this information back to the implantable medical device.

According to an embodiment of the invention, external device 20 is a stationary device with unchanging position and orientation in a room. The position of the stationary devie serves as reference for implantable device 10 in order to choose the most suitable antenna 11a or 11b for data transmission with external device 20. According to another embodiment of the invention, external device 20 is not stationary, i.e. the position of external device 20 is variable. In this case, external device 20 and implantable device 10 exchange data regarding their position and/or orientation in order to select the most suitable antenna 11a, 11b prior to radio signal data transmission.

The dependence of the coupling between two antennas, say a transmitting antenna 11 and a receiving antenna 21, that is utilized by the present invention is demonstrated in Fig. 1, which indicates that the coupling between said two antennas 11, 21 as shown in the graph on the left-hand side of Fig. 1 depends on the spatial orientations of the two antennas 11, 21 with respect to one another. In this regard, the upper part of the right-hand side of Fig. 1 shows the two antennas 11, 21 having aligned polarizations, which yields a better coupling than in the case where the two polarizations are oriented in different directions (lower part of the right-hand side of Fig. 1).

According to a further embodiment of the present invention shown in Fig. 3, the first device 10 can also be a medical device that is configured to be arranged on the patient P (e.g. on the skin of the patient). Such devices 10 are often referred to as patches. Here the first device 10 may comprise a chest belt 13 that can be fastened to the chest of the patient P. Also here, the medical device (e.g. patch) 10 comprises at least two independent antennas 11a, 11b as well as a sensor 12 configured to measure a spatial orientation of the medical device 10 with respect to e.g. an external (second) device 20 that may be used to record data transmitted by the first device 10 via radio signals C. In case the spatial orientation of the patch 10 now changes relative to the external device 20, again the most suitable antenna 11a, 11b of the first device 10 is selected - as before - based on the output signal of the sensor 12 which is indicative of said spatial orientation of the first device 10.

Finally, Fig. 4 shows further embodiments of the present invention, wherein additionally - besides determining the spatial orientation of the respective medical device 10 - the external (e.g. mobile) device 20 comprises a sensor 22 that is configured to determine the spatial orientation of the external device 20, wherein a corresponding output signal of the sensor 22 that is indicative of the spatial orientation of the external device 20 is also used to select one of said antennas 11a, 11b of the medical device 10 or to control said antennas 11a, 11b. Alternatively, or in addition, said output signal of the sensor 22 of the second device 20 may be used to select an antenna 21a, 21b of a plurality of antennas of the external device 20 so as to improve a coupling between an active antenna 11a, 11b of the medical device 10 and an active antenna 21a, 21b of the external device 20.

In the embodiments described above, the respective device 10, 20 that comprises at least two antennas 11a, 11b or 21a, 21b may also comprise more than two antennas between which the respective device may choose, e.g. for improving radio communication based on a spatial orientation of the respective device 10, 20.

## Claims

1. System, comprising:
- a first device (10) having at least two antennas (11a, 11b) for transmitting and/or receiving a radio signal,
**characterized in that**
the first device (10) comprises a sensor (12) that is configured to generate an output signal indicative of a spatial orientation of the first device (10), wherein the first device (10) is configured to use the output signal to select one of said antennas (11a, 11b) or to control said antennas (11a, 11b) for receiving a radio signal (C) and/or for transmitting a radio signal (C).

2. System according to claim 1, **characterized in that** the system (1) comprises a second device (20) configured to transmit a radio signal to the first device (10) and/or to receive a radio signal from the first device (10).

3. System according to claim 2, **characterized in that** the first device (10) is configured to select the antenna (11a, 11b) of the at least two antennas (11a, 11b) having the highest coupling with respect to at least one antenna (21a, 21b) of the second device (20).

4. System according to one of the preceding claims, **characterized in that** the at least two antennas (11a, 11b) of the first device (10) each comprise an antenna polarization, wherein said antenna polarizations are different with respect to each other.

5. System according to claim 4, **characterized in that** said antenna polarizations are orthogonal with respect to each other.

6. System according to one of the preceding claims, **characterized in that** the at least two antennas (11a, 11b) of the first device (10) are arranged at different locations.

7. System according to one of the claims 2 to 6, **characterized in that** the first device (10) is configured to control the at least two antennas (11a, 11b) of the first device (10) in a phase-controlled manner in order to increase a coupling between said at least two antennas (11a, 11b) of the first device (10) and at least one antenna (21a, 21b) of the second device (20).

8. System according to one of the claims 2 to 7, **characterized in that** the second device (20) comprises a sensor (22) that is configured to generate an output signal indicative of a spatial orientation of the second device (20).

9. System according to claim 8, **characterized in that** the second device (20) is configured to use said output signal of the sensor (22) of the second device (20) to select an antenna (21a, 21b) of at least two antennas (21a, 21b) of the second device (20) for receiving a radio signal from the first device (10) and/or for transmitting a radio signal to the first device (10).

10. System according to claim 8 or 9, **characterized in that** the system (1) is configured to also use said output signal of the sensor (22) of the second device (20) to select one of said antennas (11a, 11b) of the first device (10) or to control said antennas (11a, 11b) of the first device (10) for receiving a radio signal and/or for transmitting a radio signal.

11. System according to one of the preceding claims, **characterized in that** the first device (10) is a medical device configured to be implanted into a patient (P) or to be arranged on a patient (P).

12. System according to one of the claims 2 to 11, **characterized in that** the second device (20) is an external device.

13. System according to one of the claims 2 to 10, **characterized in that** the first device (10) is an external device.

14. System according to one of the claims 2 to 10 or according to claim 13, **characterized in that** the second device (10) is a medical device configured to be implanted into a patient (P) or to be arranged on a patient (P).

15. System according claim 11 or 14, **characterized in that** the medical device (10) is attachable to the patient and comprises a patch, or wherein the medical device (10) can be worn by the patient and comprises a chest belt (13), a belt, a watch, eyeglasses, a headband or any clothing item.
